# EUROPEAN PATENT APPLICATION

(11) **EP 4 151 169 A1**
(43) Date of publication of application: **22.03.2023**
(21) Application number: 22195729.3
(22) Date of filing: 14.09.2022
(51) Int. Cl.: A61B 18/14, A61B 34/30

(54) **VESSEL SEALER WITH SMART CUTTING**

(30) Priority: 15.09.2021 US 202163244306 P; 18.08.2022 US 202217890447
(71) Applicant: Covidien LP, Mansfield, MA 02048 (US)
(72) Inventor: MCHENRY, Jennifer R., Boulder, 80301 (US); SIMS, Grant T., Boulder, 80301 (US); JOSEPH, Daniel A., Boulder, 80301 (US)
(74) Representative: Maschio, Antonio

(57) **Abstract**

A knife limit for a surgical instrument includes a housing having a shaft extending therefrom configured to support an end effector at a distal end thereof, the end effector including first and second jaw members. One or both of the jaw members including a knife channel defined therein and extending therealong to a distal portion thereof. A knife assembly is disposed within the housing and cooperates with a trigger to translate a knife within the knife channel to the distal portion of the jaw member upon actuation thereof. A knife limit button is disposed within the housing and is configured to limit the distal translation of the knife within the knife channel upon selective actuation thereof. The knife limit button is movable between a first position allowing full translation of the knife within the knife channel and a second position limiting distal translation of the knife within the knife channel.

## Description

### CROSS REFERENCE TO RELATED APPLICATION

This application claims the benefit of U.S. Provisional Application Serial No. 63/244,306 filed September 15, 2021, the entire contents of which being incorporated by reference herein.

### FIELD

The present disclosure relates to surgical instruments and, more specifically, to sealing instruments such as, for example, for use in endoscopic instruments and robotic surgical systems, and methods relating to the same.

### BACKGROUND

Electrosurgical forceps utilize both mechanical clamping action and electrical energy to affect hemostasis by heating the tissue and blood vessels to coagulate, cauterize and/or seal tissue.

As is known, in order to effectively seal larger vessels (or tissue) two predominant mechanical parameters are accurately controlled - the pressure applied to the vessel (tissue) and the gap distance between the electrodes - both of which are affected by the thickness of the sealed vessel. More particularly, accurate application of pressure is important to oppose the walls of the vessel; to reduce the tissue impedance to a low enough value that allows enough electrosurgical energy through the tissue; to overcome the forces of expansion during tissue heating; and to contribute to the end tissue thickness which is an indication of a good seal.

As mentioned above, in order to properly and effectively seal larger vessels or tissue, a greater closure force between opposing jaw members is required especially to ensure sealing at the tip of the surgical instrument. If a seal at or proximate the tip of the jaw members is not successful or even marginally successful, cutting of the tissue may be compromised.

With conventionally endoscopic forceps, various methods and algorithms have been developed that may sense seal integrity at or proximate the tip of the jaw members and warn the user of the condition thereof. With robotic assisted surgery or endoscopic visualization systems, the integrity of the seal at or proximate the tip of the jaw members many be more difficult to determine. As a result thereof, it would desirous to develop a vessel sealing instrument or robotic assisted sealing instrument that shortens the length of the cut based upon one or more thresholds or parameters being set.

### SUMMARY

As used herein, the term "distal" refers to the portion that is being described which is further from an operator (whether a human surgeon or a surgical robot), while the term "proximal" refers to the portion that is being described which is closer to the operator. The terms "about," substantially," and the like, as utilized herein, are meant to account for manufacturing, material, environmental, use, and/or measurement tolerances and variations. Further, to the extent consistent, any of the aspects described herein may be used in conjunction with any or all of the other aspects described herein. Moreover, rotation may be measure in degrees or radians.

Provided in accordance with aspects of the present disclosure is a knife limit for a surgical instrument that includes a housing having a shaft extending therefrom configured to support an end effector at a distal end thereof, the end effector including first and second jaw members. One or both of the jaw members is moveable relative to the other jaw member to grasp tissue therebetween. One or both of the jaw members includes a knife channel defined therein and that extends therealong from a proximal portion to a distal portion of the jaw member. A knife assembly is disposed within the housing and cooperates with a trigger disposed on the housing to translate a knife within the knife channel to the distal portion of the jaw member upon actuation thereof.

A knife limit button is operably disposed within the housing and is configured to limit the distal translation of the knife within the knife channel upon selective actuation thereof. The knife limit button is selectively movable between a first position allowing full translation of the knife within the knife channel to the distal portion upon actuation of the trigger and one or more additional positions limiting distal translation of the knife within the knife channel to a position proximal to the distal portion.

In aspects according to the present disclosure, the knife limit button is moveable to a second position wherein the knife limit button limits distal translation of the knife assembly which, in turn, limits distal translation of the knife within the knife channel.

In aspects according to the present disclosure, the knife limit button is moveable to a second position wherein the knife limit button limits proximal translation of the trigger which, in turn, limits distal translation of the knife within the knife channel.

In aspects according to the present disclosure, the knife limit button is rotatable to a second position wherein the knife limit button limits distal translation of the knife assembly which, in turn, limits distal translation of the knife with the knife channel.

In aspects according to the present disclosure, the knife assembly includes a cuff that rides atop the shaft and wherein upon actuation of the knife limit button the cuff is prevented from fully translating atop the shaft. In other aspects according to the present disclosure, the knife assembly includes a cuff that rides atop the shaft and wherein upon rotation of the knife limit button the cuff is prevented from fully translating atop the shaft.

In aspects according to the present disclosure, the knife limit communicates with a sensor operably coupled to the housing, the sensor configured to cooperate with the knife limit button to limit distal translation of the knife.

In aspects according to the present disclosure, the knife limit communicates with a sensor operably associated with the housing or a source of electrosurgical energy, the sensor configured to notify a user of the surgical instrument to actuate the knife limit button to limit distal translation of the knife.

Provided in accordance with aspects of the present disclosure is a method of limiting distal translation of a knife blade of a robotic surgical instrument, including selectively engaging an end effector onto a housing of a robotic surgical instrument and coupling the end effector to a jaw drive input, the end effector including first and second jaw members, one or both of the first or second jaw members is moveable relative to the other jaw member to grasp tissue therebetween, one or both of the first or second jaw members including a knife channel defined therein and extending therealong from a proximal portion to a distal portion of the jaw member.

The method further includes: communicating with the end effector to recognize the end effector and associated operating parameters and characteristics therewith and communicating operational data back to an EPROM or PCB, the operational characteristics including a length of the knife channel defined within the jaw members of the end effector assembly; initiating a homing algorithm to determine a fully retracted or home position of a knife blade disposed within the knife channel between the jaw members and calculating a number of rotations of a knife drive coupler to fully translate the knife to the distal portion of the knife channel; and selectively actuating a knife limit button to limit the number of rotations of the knife drive coupler to limit distal translation of the knife within the knife channel.

In aspects according to the present disclosure, the method further includes selectively actuating the knife limit button based on feedback from a tissue sensor. In other aspects according to the present disclosure, the knife limit button operably communicates with a tissue sensor disposed within the housing or an electrosurgical energy source to determine automatic actuation of the knife limit button.

In aspects according to the present disclosure, the knife limit button operably communicates with a tissue sensor disposed within the housing or an electrosurgical energy source to alert a surgeon to actuate the knife limit button. In other aspects according to the present disclosure, the tissue sensor is configured to determine the quality of a tissue seal between jaw members.

In aspects according to the present disclosure, the tissue sensor is configured to determine the quality of a tissue seal between jaw members based on at least one of the angular position of the jaw members, the tissue thickness, the activation time of the electrosurgical energy, or end tissue thickness after seal.

### BRIEF DESCRIPTION OF THE DRAWINGS

Various aspects and features of the present disclosure are described hereinbelow with reference to the drawings wherein like numerals designate identical or corresponding elements in each of the several views.
FIG. 1A is a perspective view of a bipolar forceps shown in open configuration and including a housing, a shaft, handle assembly, trigger assembly and an end effector assembly according to the present disclosure;
FIG. 1B is a perspective view of the bipolar forceps of FIG. 1A shown in closed configuration;
FIG. 2 is a rear view of the forceps of FIG. 1A;
FIG. 3A is an enlarged, front perspective view of the end effector assembly of FIG. 1A shown in an open configuration;
FIG. 3B is an enlarged, front perspective view of the end effector assembly of FIG. 1A shown in a closed configuration;
FIG. 3C is an enlarged, side view of the end effector assembly of FIG. 1A shown in open configuration;
FIGS. 4A-4C are internal views of another embodiment of a bipolar forceps including a knife limit button and actuation thereof;
FIGS. 4D-4E are internal views of additional embodiments knife limit buttons;
FIGS. 5-7 are various perspective views of a robotic surgical instrument provided in accordance with the present disclosure configured for mounting on a robotic arm of a robotic surgical system and including a knife limit feature; and
FIG. 8 is a schematic illustration of an exemplary robotic surgical system configured to releasably receive the surgical instrument of FIGS. 5-7.

### DETAILED DESCRIPTION

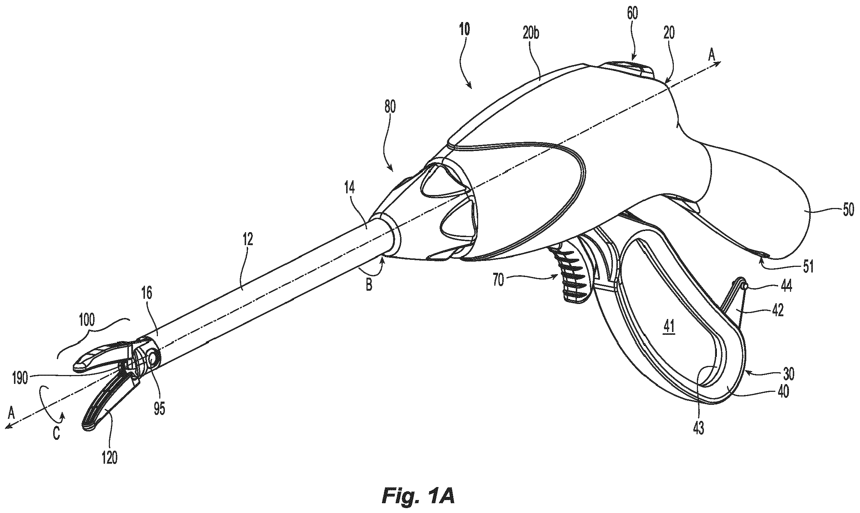

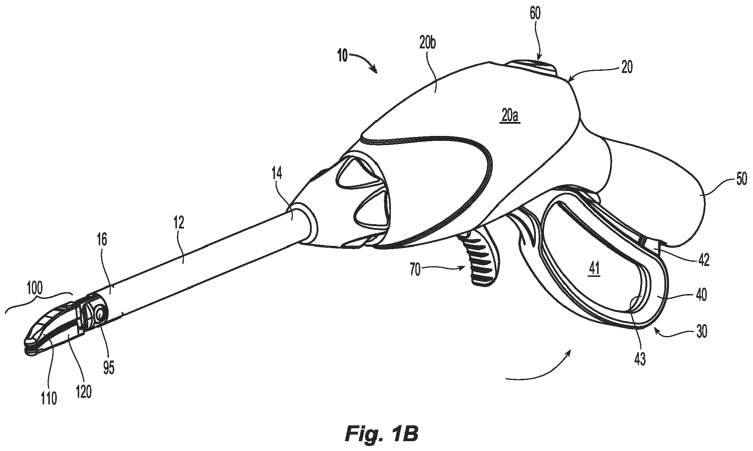

Turning now to FIGS. 1A-2, one embodiment of an endoscopic bipolar forceps 10 is shown for use with various surgical procedures and generally includes a housing 20, a handle assembly 30, a rotating assembly 80, a trigger assembly 70 and an end effector assembly 100 which mutually cooperate to grasp, seal and divide large tubular vessels and large vascular tissues. Although the majority of the figure drawings depict a bipolar forceps 10 for use in connection with endoscopic surgical procedures, the present disclosure may be used for more traditional open surgical procedures. For the purposes herein, the forceps 10 is described in terms of an endoscopic instrument, however, it is contemplated that an open version of the forceps may also include the same or similar operating components and features as described below.

Forceps 10 includes a shaft 12 which has a distal end 16 dimensioned to mechanically engage the end effector assembly 100 and a proximal end 14 which mechanically engages the housing 20. Details of how the shaft 12 connects to the end effector are described in more detail below with respect to FIGS. 13 and 14. The proximal end 14 of shaft 12 is received within the housing 20 and the connections relating thereto are also described in detail below with respect to FIGS. 11 and 12. In the drawings and in the descriptions which follow, the term "proximal," as is traditional, will refer to the end of the forceps 10 which is closer to the user, while the term "distal" will refer to the end which is farther from the user.

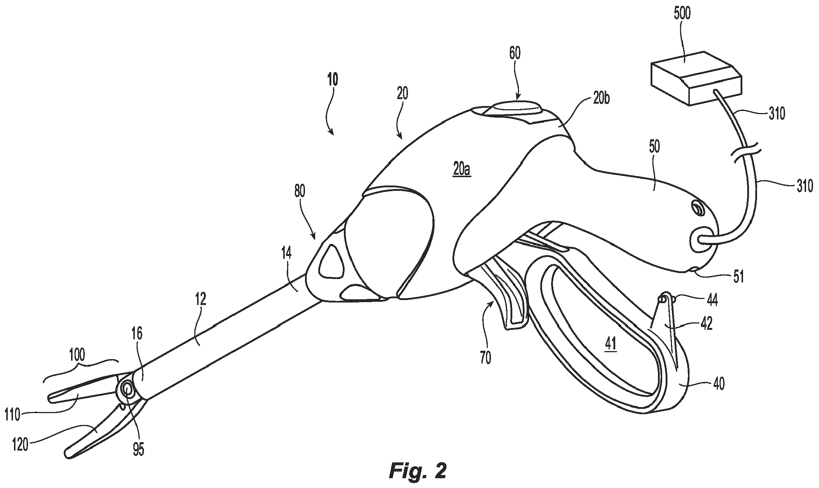

As best seen in FIGS. 1A and 2, forceps 10 also includes an electrosurgical cable 310 which connects the forceps 10 to a source of electrosurgical energy, e.g., a generator 500 (shown schematically. Generator 500 may include various safety and performance features including isolated output, independent activation of accessories.

Handle assembly 30 includes a fixed handle 50 and a movable handle 40. Fixed handle 50 is integrally associated with housing 20 and handle 40 is movable relative to fixed handle 50 as explained in more detail below with respect to the operation of the forceps 10. Fixed handle 50 is oriented approximately 30 degrees relative a longitudinal axis "A-A defined through shaft 12. Fixed handle 50 may include one or more ergonomic enhancing elements to facilitate handling, e.g., scallops, protuberances, elastomeric material, etc.

Rotating assembly 80 is operatively associated with the housing 20 and is rotatable approximately 180 degrees about a longitudinal axis "A-A" (See FIG. 1A).

As mentioned above, end effector assembly 100 is attached at the distal end 14 of shaft 12 and includes a pair of opposing jaw members 110 and 120. Movable handle 40 of handle assembly 30 is ultimately connected to a drive assembly (not shown) which, together, mechanically cooperate to impart movement of the jaw members 110 and 120 from an open position wherein the jaw members 110 and 120 are disposed in spaced relation relative to one another, to a clamping or closed position wherein the jaw members 110 and 120 cooperate to grasp tissue therebetween.

Turning now to the more detailed features of the present disclosure, movable handle 40 includes a finger loop 43 which has an aperture 41 defined therethrough which enables a user to grasp and move the handle 40 relative to the fixed handle 50. Finger loop 43 is typically ergonomically enhanced and may include one or more gripping elements (not shown) disposed along the inner peripheral edge of aperture 41 which are designed to facilitate gripping of the movable handle 40 during activation, e.g., a so called "soft touch" material. Gripping elements may include one or more protuberances, scallops and/or ribs to enhance gripping.

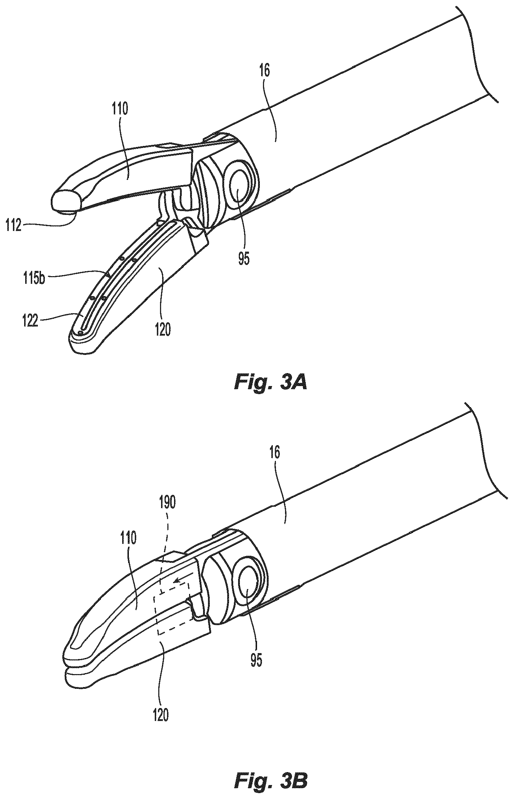

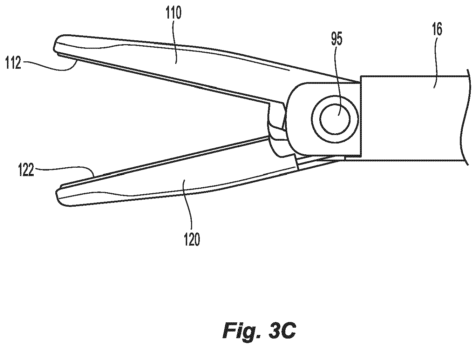

As shown best in FIGS. 3A-3C, the end effector assembly 100 includes opposing jaw members 110 and 120 which cooperate to effectively grasp tissue for sealing purposes. The end effector assembly 100 is designed as a bilateral assembly, i.e., both jaw members 110 and 120 pivot relative to one another about a pivot pin 95 disposed therethrough. The jaw members 110 and 120 are curved to facilitate manipulation of tissue and to provide better "line of sight" for accessing organs and large tissue structures.

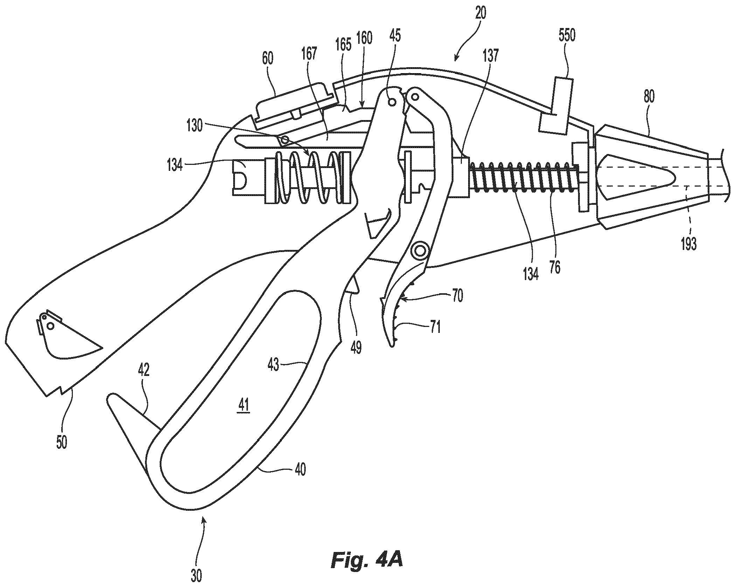

A reciprocating drive sleeve 134 is slidingly disposed within the shaft 12 and is remotely operable by the drive assembly 130 (FIG. 4A). Trigger assembly 70 cooperates with the knife assembly 160 to selectively translate knife 190 (FIG. 3B) through a tissue seal. The knife assembly 160 includes a reciprocating knife bar 167 which mounts atop the drive sleeve 134. Knife bar 167 includes a cuff 137 disposed at the distal end thereof. Cuff 137 is dimensioned to encapsulate drive sleeve 134 when the knife assembly 160 is assembled. A spring 76 biases the cuff in a proximal-most orientation. A knife carriage 165 mounts to the upper end of the finger actuator 71 of the trigger 70 assembly.

When the handle 40 is disposed in a spaced-apart or open configuration relative to handle 50, a flange 49 which extends from handle 40 prevents actuation of the trigger assembly 70. More particularly, finger actuator 71 is prevented from being actuated proximally by flange 49 when the jaw members 110 and 120 are open. As can be appreciated, this prevents premature actuation of the knife 190 when tissue is not grasped between jaw members 110 and 120. When handle 40 is selectively moved relative to handle 50, a gap is formed between the flange 49 and the finger actuator 71 and the user is free to selectively actuate the knife 190 by squeezing the finger actuator 71 proximally FIG. 4A.

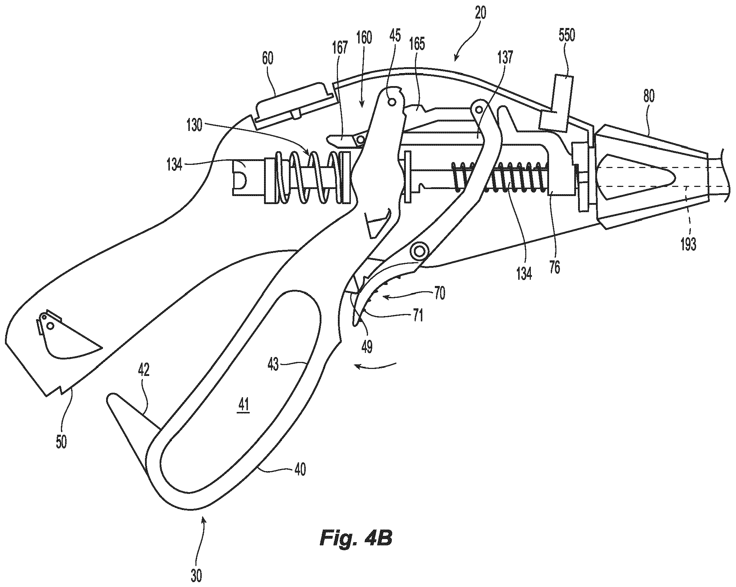

Once the clearance is provided by movement of handle 40, proximal movement of the finger actuator 71 results in distal translation of the knife bar 167 and internally disposed knife rod 193 and knife 190 (FIG. 4B). Upon release of finger actuator 71, spring 76 biases the knife 190 back to a proximal-most position. Detail relating to the movement of the knife assembly are discussed with reference to U.S. Patent No. 7,766,910 the entire contents of which are incorporated by reference herein.

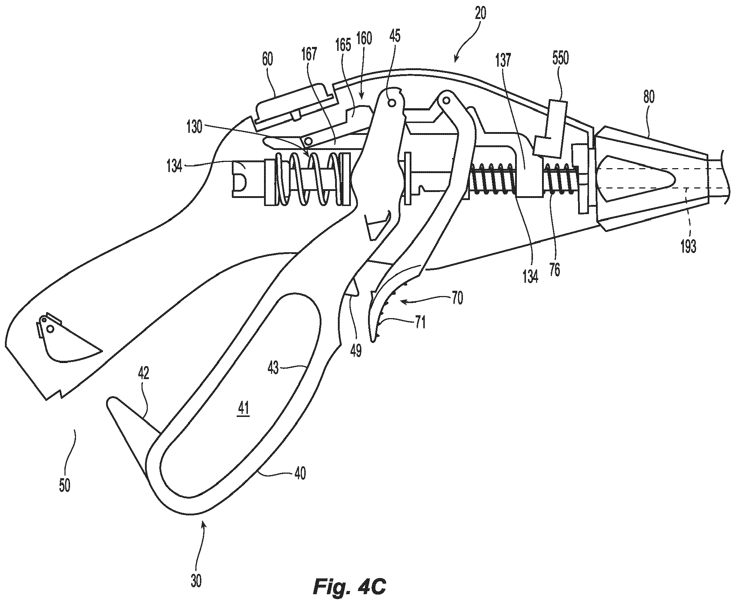

FIG. 4A-4C also show a knife limit button 550 which is designed to limit the extent the knife 190 extends between jaw members 110, 120 for depending upon a particular purpose. More particularly, knife limit button 550 is disposed within the housing 20 and is selectively positionable between a first inactive position (FIG. 4A) and a second deployed position for limiting knife travel (FIG. 4C). The button 550 may be limited to two positions, e.g., inactive or full deployed, or the button may be configured with multiple levels of activation depending on a particular purpose, e.g., inactive (knife travels fully between jaw members 110, 120), limited deployment (knife only travels three-quarters between jaw members 110, 120), full deployment (knife travels halfway between jaw members 110, 120).

During use, when a surgeon is grasping tissue, e.g., a large tissue bundle, the tissue proximate the tip may be prone for incomplete sealing due to the relative and angular position of the jaw members 110, 120, the inability of the generator to supply additional energy to complete the seal at the tip, or insufficient gap between the jaw members. As a result, the surgeon, when actuating the knife 190, may not want the knife 190 to fully deploy to the tip until the surgeon is able to regrasp the tissue with a more proximal portion of the jaw members 110, 120 to complete the tissue seal. Without the ability to see the knife 190 during actuation and translation, it may prove difficult to properly estimate the knife travel desired. By actuating the knife limit button 550, the surgeon can fully actuate the trigger 70 and the knife travel will automatically be limited in accordance with the parameters of the knife lockout button 550 or the position of the knife lockout button 550 (See FIG. 4C).

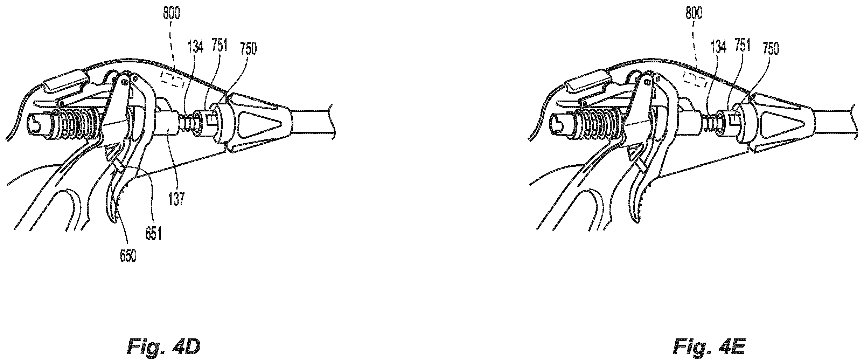

FIGS. 4D and 4E show other embodiments of knife lockouts 650, 750, respectively, for use with instrument 10. Details relating to instrument 10 are described above and, as such, only those elements pertinent to the operation of lockouts 650 and 750 are discussed below. Both lockouts 650 and 750 are configured to rely on a feedback mechanism, visual monitoring system or sensor 800 that operably communicates with the jaw members 110, 120 (or generator 500) to enable the so-called "short cut" feature of the knife 190 (e.g., activate the knife lockout 650 or 750).

Knife lockout 650 includes a stop or pin 651 (or other obstruction) that is selectively moveable within the path of the trigger 70 to prevent the trigger 70 from fully actuating. Pin 651 operably communicates with the sensor 800 to deploy when full transection of the vessel or tissue disposed between the jaw members 110, 120 is undesirous. Pin 651 may be manually deployable as well. Pin 651 may be selectively extendible to one or more lengths to differ the stroke length of the knife 190 depending upon a particular purpose, e.g., % stroke or ½ stroke.

Knife lockout 750 includes a stop block 751 (or other obstruction) that is selectively moveable along drive sleeve 134 within the path of cuff 137 to prevent the cuff 137 from fully advancing distally. Stop block 751 communicates with the sensor 800 to deploy when full transection of the vessel or tissue disposed between the jaw members 110, 120 is undesirous. Stop block 751 may also be manually deployable. For example, stop block 751 may be automatically rotatable upon request by the sensor 800 to rotate and block full distal translation of the cuff 137. Stop block 751 may be manually selectively extendible (e.g., proximally in the path of cuff 137) to one or more positions to differ the stroke length of the knife 190 depending upon a particular purpose, e.g., ¾ stroke or ½ stroke. In embodiments, the cuff 137 may be grounded within the housing 20 and when actuated simply extends to prevent further translation. Other embodiments envision operably associating the stop block 751 with the rotation wheel to prevent distal translation of the cuff 137.

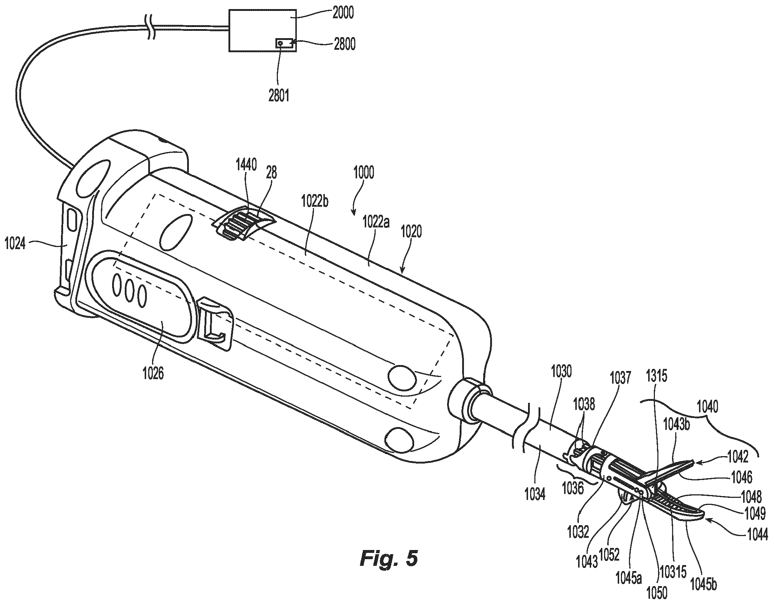

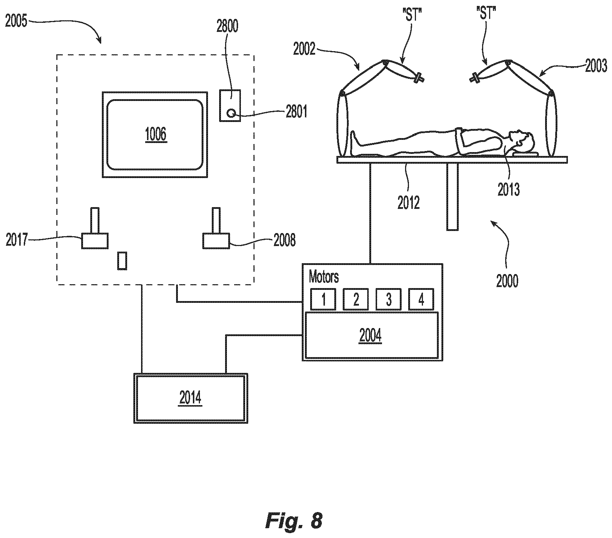

The presently disclosed knife lockout button may be employed with a robotic instrument such as the robotic surgical instrument 1000 shown in FIGS. 5-8. Surgical instrument 1000 provided in accordance with the present disclosure generally includes a housing 1020, a shaft 1030 extending distally from housing 1020, an end effector assembly 1040 extending distally from shaft 1030, and an actuation assembly 1100 disposed within housing 1020 and operably associated with end effector assembly 1040. Instrument 1010 is detailed herein as an articulating electrosurgical forceps configured for use with a robotic surgical system, e.g., robotic surgical system 2000 (FIG. 8).

Turning briefly to FIG. 8, robotic surgical system 2000 is configured for use in accordance with the present disclosure. Aspects and features of robotic surgical system 2000 not germane to the understanding of the present disclosure are omitted to avoid obscuring the aspects and features of the present disclosure in unnecessary detail.

Robotic surgical system 2000 generally includes a plurality of robot arms 2002, 2003; a control device 2004; and an operating console 2005 coupled with control device 2004. Operating console 2005 may include a display device 2006, which may be set up in particular to display three-dimensional images; and manual input devices 2007, 2008, by means of which a person, e.g., a surgeon, may be able to telemanipulate robot arms 2002, 2003 in a first operating mode. Robotic surgical system 2000 may be configured for use on a patient 2013 lying on a patient table 2012 to be treated in a minimally invasive manner. Robotic surgical system 2000 may further include a database 2014, in particular coupled to control device 2004, in which are stored, for example, pre-operative data from patient 2013 and/or anatomical atlases.

Each of the robot arms 2002, 2003 may include a plurality of members, which are connected through joints, and a mounted device which may be, for example, a surgical tool "ST." One or more of the surgical tools "ST" may be instrument 1000 (FIG. 5), thus providing such functionality on a robotic surgical system 2000.

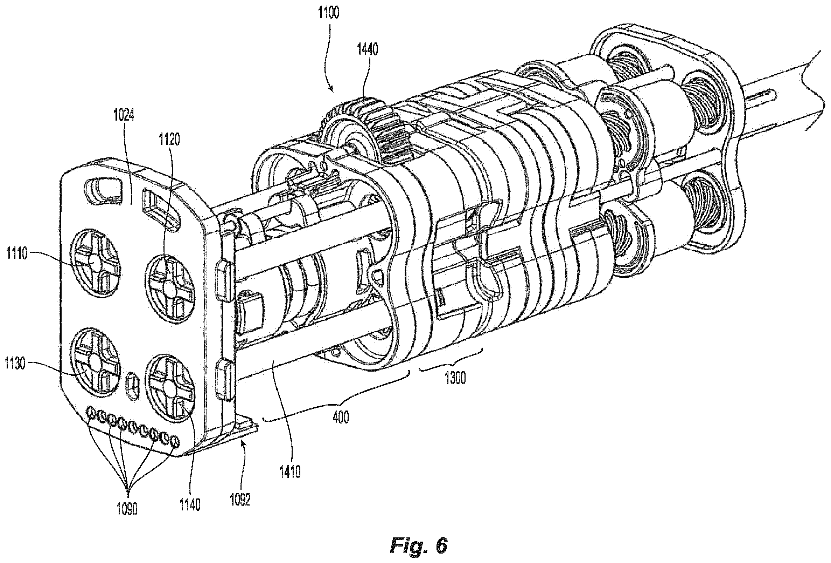

Robot arms 2002, 2003 may be driven by electric drives, e.g., motors, connected to control device 2004. The motors, for example, may be rotational drive motors configured to provide rotational inputs, e.g., to selectively rotationally drive input couplers 1110-1140 (FIG. 6) of surgical instrument to accomplish a desired task or tasks. Control device 2004, e.g., a computer, may be configured to activate the motors, in particular by means of a computer program, in such a way that robot arms 2002, 2003, and, thus, their mounted surgical tools "ST" execute a desired movement and/or function according to a corresponding input from manual input devices 2007, 2008, respectively. Control device 2004 may also be configured in such a way that it regulates the movement of robot arms 2002, 2003 and/or of the motors.

Control device 2004, more specifically, may control one or more of the motors based on rotation, e.g., controlling to rotational position using a rotational position encoder (or Hall effect sensors or other suitable rotational position detectors) associated with the motor to determine a degree of rotation output from the motor and, thus, the degree of rotational input provided to the corresponding input coupler 1110-1140 (FIG. 6) of surgical instrument 1000). Alternatively or additionally, control device 2004 may control one or more of the motors based on torque, current, or in any other suitable manner.

With particular reference to FIG. 5, housing 1020 of instrument 1000 includes first and second body portion 1022a, 1022b and a proximal face plate 1024 that cooperate to enclose actuation assembly 1100 therein. Proximal face plate 1024 includes apertures defined therein through which input couplers 1110-1140 (FIG. 6) of actuation assembly 1100 extend. A pair of latch levers 1026 (only one of which is illustrated in FIG. 5) extending outwardly from opposing sides of housing 1020 enable releasable engagement of housing 1020 with a robotic arm of a surgical system, e.g., robotic surgical system 2000 (FIG. 8). Thumbwheel 1440 extends through housing 1020 to enable manual manipulation of thumbwheel 1440 from the exterior of housing 1020 to permit manual opening and closing of end effector assembly 1040.

A plurality of electrical contacts 1090 extend through one or more apertures defined through proximal face plate 1024 to enable electrical communication between instrument 1000 and robotic surgical system 2000 (FIG. 8) when instrument 1000 is engaged thereon, e.g., for the communication of data, control, and/or power signals therebetween. As an alternative to electrical contacts 1090 extending through proximal face plate 1024, other suitable transmitter, receiver, and/or transceiver components to enable the communication of data, control, and/or power signals are also contemplated, e.g., using RFID, Bluetooth^{®}, WiFi^{®}, or via any other suitable wired, wireless, contacted, or contactless communication method. At least some of the electrical contacts 1090 are electrically coupled with electronics 1092 mounted on an interior side of proximal face plate 1024, e.g., within housing 1020. Electronics 1092 may include, for example, a storage device, a communications device (including suitable input/output components), and a CPU including a memory and a processor. Electronics 1092 may be mounted on a circuit board or otherwise configured, e.g., as a chip.

The storage device of electronics 1092 stores information relating to surgical instrument such as, for example: the item number, e.g., SKU number; date of manufacture; manufacture location, e.g., location code; serial number; lot number; use information; setting information; adjustment information; calibration information; security information, e.g., encryption key(s), and/or other suitable additional or alternative data. The storage device of electronics 1092 may be, for example, a magnetic disk, flash memory, optical disk, or other suitable data storage device.

As an alternative or in addition to storing the above-noted information in the storage device of electronics 1092, some or all of such information, e.g., the use information, calibration information, setting information, and/or adjustment information, may be stored in a storage device associated with robotic surgical system 2000 (FIG. 8), a remote server, a cloud server, etc., and accessible via instrument 1000 and/or robotic surgical system 2000 (FIG. 8). In such configurations, the information may, for example, be updated by manufacturer-provided updates, and/or may be applied to individual instruments, units of instruments (e.g., units from the same manufacturing location, manufacturing period, lot number, etc.), or across all instruments. Further still, even where the information is stored locally on each instrument, this information may be updated by manufacturer-provided updates manually or automatically upon connection to the robotic surgical system 2000 (FIG. 8).

Referring again to FIG. 5, shaft 1030 of instrument 1000 includes a distal segment 1032, a proximal segment 1034, and an articulating section 1036 disposed between the distal and proximal segments 1032, 1034, respectively. Articulating section 1036 includes one or more articulating components 1037, e.g., links, joints, etc. A plurality of articulation cables 1038, e.g., four (4) articulation cables, or other suitable actuators, extend through articulating section 1036. More specifically, articulation cables 1038 are operably coupled to distal segment 1032 of shaft 1030 at the distal ends thereof and extend proximally from distal segment 1032 of shaft 1030, through articulating section 1036 of shaft 1030 and proximal segment 1034 of shaft 1030, and into housing 1020, wherein articulation cables 1038 operably couple with an articulation sub-assembly 1200 of actuation assembly 1100 to enable selective articulation of distal segment 1032 (and, thus end effector assembly 1040) relative to proximal segment 1034 and housing 1020, e.g., about at least two axes of articulation (yaw and pitch articulation, for example). Articulation cables 1038 are arranged in a generally rectangular configuration, although other suitable configurations are also contemplated. In some configurations, as an alternative, shaft 1030 is substantially rigid, malleable, or flexible and not configured for active articulation.

With respect to articulation of end effector assembly 1040 relative to proximal segment 1034 of shaft 1030, actuation of articulation cables 1038 may be accomplished in pairs. More specifically, in order to pitch end effector assembly 1040, the upper pair of cables 1038 are actuated in a similar manner while the lower pair of cables 1038 are actuated in a similar manner relative to one another but an opposite manner relative to the upper pair of cables 1038. With respect to yaw articulation, the right pair of cables 1038 are actuated in a similar manner while the left pair of cables 1038 are actuated in a similar manner relative to one another but an opposite manner relative to the right pair of cables 1038. Other configurations of articulation cables 1038 or other articulation actuators are also contemplated.

Continuing with reference to FIG. 5, end effector assembly 1040 includes first and second jaw members 1042, 1044, respectively. Each jaw member 1042, 1044 includes a proximal flange portion 1043a, 1045a and a distal body portion 1043b, 1045b, respectively. Distal body portions 1043b, 1045b define opposed tissue-contacting surfaces 1046, 1048, respectively. Proximal flange portions 1043a, 1045a are pivotably coupled to one another about a pivot 1050 and are operably coupled to one another via a cam-slot assembly 1052 including a cam pin slidably received within cam slots defined within the proximal flange portion 1043a, 1045a of at least one of the jaw members 1042, 1044, respectively, to enable pivoting of jaw member 1042 relative to jaw member 1044 and distal segment 1032 of shaft 1030 between a spaced-apart position (e.g., an open position of end effector assembly 1040) and an approximated position (e.g., a closed position of end effector assembly 1040) for grasping tissue between tissue-contacting surfaces 1046, 1048. As an alternative to this unilateral configuration, a bilateral configuration may be provided whereby both jaw members 1042, 1044 are pivotable relative to one another and distal segment 1032 of shaft 1030. Other suitable jaw actuation mechanisms are also contemplated.

A longitudinally-extending knife channel 1049 (only knife channel 1049 of jaw member 1044 is illustrated; the knife channel of jaw member 1042 is similarly configured) is defined through the tissue-contacting surface 1046, 1048 of one or both jaw members 1042, 1044. In such embodiments, a knife assembly including a knife tube (not shown) extends from housing 1020 through shaft 1030 to end effector assembly 1040 and a knife blade 1315 disposed within end effector assembly 1040 between jaw members 1042, 1044 is provided. The knife blade 1315 is selectively translatable through the knife channel(s) 1049 and between the jaw member 1042, 1044 to cut tissue grasped between tissue-contacting surfaces 1046, 1048 of jaw members 1042, 1044, respectively. The knife tube is operably coupled to a knife drive sub-assembly 1300 (FIG. 6) of actuation assembly 1100 at a proximal end thereof to enable the selective actuation of the knife tube to, in turn, reciprocate the knife blade 1315 between jaw members 1042, 1044 to cut tissue grasped between tissue-contacting surfaces 1046,

Knife drive sub-assembly 1300 is operably coupled between third input coupler 1130 of actuation assembly 1100 and the knife tube such that, upon receipt of appropriate input into third input coupler 1130, knife drive sub-assembly 1300 manipulates the knife tube to reciprocate the knife blade 1315 between jaw members 1042, 1044 to cut tissue grasped between tissue-contacting surfaces 1046, 1048.

Actuation assembly 1100 is configured to operably interface with a robotic surgical system 2000 (FIG. 8) when instrument 1000 is mounted on robotic surgical system 2000 to enable robotic operation of actuation assembly 1100 to provide the above-detailed functionality. That is, robotic surgical system 2000 selectively provides inputs, e.g., rotational inputs to input couplers 1110-1140 of actuation assembly 1100 to articulate end effector assembly 1040, grasp tissue between jaw members 1042, 1044, and/or cut tissue grasped between jaw members 1042, 1044. However, it is also contemplated that actuation assembly 1100 be configured to interface with any other suitable surgical system, e.g., a manual surgical handle, a powered surgical handle, etc. Robotic surgical system 2000) is generally described with respect to U.S. Patent Application Serial No. 63/183,093 the entire contents of which being incorporated by reference herein.

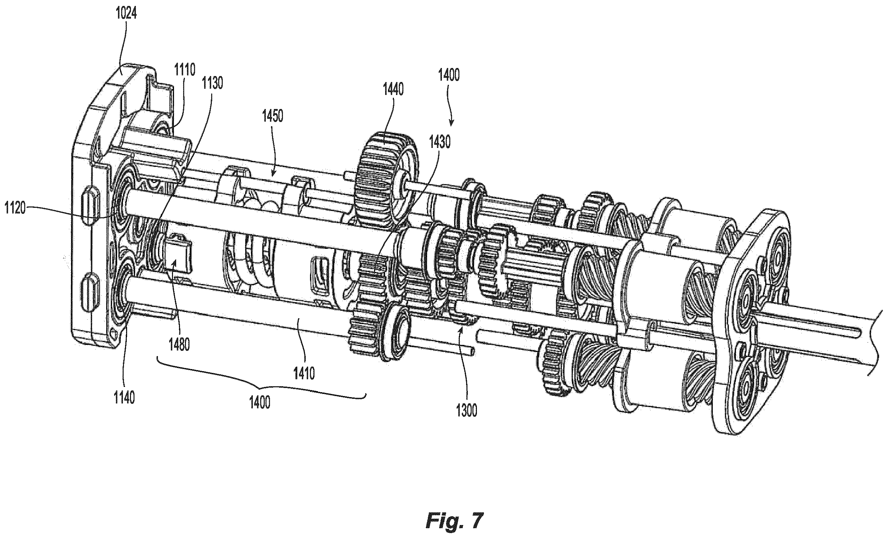

With reference to FIG. 7, jaw drive sub-assembly 1400 of actuation assembly 1100 is shown generally including an input shaft 1410, an input gear 1420, a drive gear 1430, a thumbwheel 1440, a spring force assembly 1450, and a drive rod assembly 1480. Details relating to the jaw drive sub-assembly 1400 and various calibration methods and algorithms are disclosed in U.S. Patent Application Serial No. 63/183,093 the entire contents of which being incorporated by reference herein.

With tissue grasped between jaw members 1042, 1044 under an appropriate jaw pressure, energy may be supplied to jaw members 1042, 1044 to treat, e.g., seal tissue. Thereafter, the knife blade 1315 may be advanced between jaw members 1042, 1044 to cut the treated tissue, e.g., by providing a rotational input to input coupler 1130 (FIG. 6) to actuate knife drive sub-assembly 1300 to translate the knife tube distally to thereby advance the knife blade 1315 between jaw members 1042, 1044 to cut the treated tissue. Alternatively, tissue may be cut without first treating the tissue and/or tissue may be treated without subsequent cutting. Once tissue is cut, an opposite rotation input is provided to input coupler 1130 (FIG. 6) to return the knife blade 1315 to its initial position.

As mentioned above, by actuating a knife limit button 2800 on the surgical console (FIGS. 5 and 8), the surgeon can fully actuate the knife drive sub-assembly 1300 by causing the input coupler 1130 of actuation assembly 1100 to rotate (e.g., remotely), such that, upon receipt of appropriate input into third input coupler 1130, knife drive sub-assembly 1300 manipulates the knife tube to reciprocate the knife blade 1315 between jaw members 1042, 1044 to cut tissue grasped between tissue-contacting surfaces 1046, 1048. During use, when a surgeon is grasping tissue, e.g., a large tissue bundle, the tissue proximate the tip may be prone for incomplete sealing due to the relative and angular position of the jaw members 1042, 1044, the inability of the generator to supply additional energy to complete the seal at the tip, or insufficient gap between the jaw members 1042, 4044. As a result, the surgeon, when actuating the knife 1315, may not want the knife 1315 to fully deploy to the tip until the surgeon is able to regrasp the tissue with a more proximal portion of the jaw members 1042, 1044 to complete the tissue seal. Without the ability to see the knife during actuation and translation, it may prove difficult to properly estimate the knife travel desired. By actuating a knife limit button 2800 on the surgical console (FIGS. 5 and 8), the travel of the knife 1315 is automatically controlled by adjusting the number of rotations of input coupler 1130. Knife 1315 will automatically be limited in accordance with the parameters of the knife lockout button 550 or the position of the knife lockout button 550 (See FIG. 4C). In embodiments, the knife lockout button (or other knife lockouts described above) may be configured to reduce the length of the knife travel by a percentage regardless of jaw size, e.g., in the range of about 20-30%.

In embodiments an algorithm may be employed to actuate the knife limit button 2800 (or it may be internally controlled by the algorithm) based on feedback from the actual tissue seal or the relative position of the jaw members 1042, 1044. More particularly, if the algorithm receives feed back relating to the tissue seal (e.g., tissue seal quality at the tip of the jaw members 1042, 1044), the travel of the knife 1315 may automatically adjust the number or rotations of input coupler 1130 (via knife limit button 280 or internally within algorithm) to reduce a possible tissue bleed or incomplete tissue cut. Moreover, the algorithm may receive feedback relating to the relative position of the jaw members 1042, 1044 (e.g., relative to one another, angular position, gap, etc.) and may automatically adjust the number or rotations of input coupler 1130 (via knife limit button 2800 or internally within algorithm) to reduce a possible tissue bleed or incomplete tissue cut. The knife limit button 2800 or the algorithm may automatically differ the stroke length of the knife 1315 depending upon a particular purpose, e.g., ¾ stroke or ½ stroke. An indicator 2801 may be operably associated with the knife limit button 2800 or algorithm to alert the surgeon of a short cut condition.

The present disclosure also discloses a method of limiting distal translation of a knife 1315 of a robotic surgical instrument 1000, including selectively engaging an end effector 1040 onto a housing 1020 of a robotic surgical instrument 1000 and coupling the end effector 1040 to a jaw drive input 10. The end effector 1040 includes first and second jaw members 1042, 1044, respectively, one or both of the first or second jaw members, e.g., jaw member 1042 is moveable relative to the other jaw member 1044 to grasp tissue therebetween, one or both of the first or second jaw members, e.g., jaw member 1044, including a knife channel 1049 defined therein and extending therealong from a proximal portion to a distal portion of the one jaw member.

The method further includes: communicating with the end effector 1040 to recognize the end effector 1040 and associated operating parameters and characteristics therewith and communicating operational data back to an EPROM or PCB, the operational characteristics including a length of the knife channel 1049 defined within the jaw member 1044, of the end effector assembly 1040; initiating a homing algorithm to determine a fully retracted or home position of a knife blade 1315 disposed within the knife channel 1049 between the jaw members 1042, 1044 and calculating a number of rotations of a knife drive coupler 1130 to fully translate the knife 1315 to the distal portion of the knife channel 1049; and selectively actuating a knife limit button 2800 to limit the number of rotations of the knife drive coupler to 1130 limit distal translation of the knife 1315 within the knife channel 1049.

While several embodiments of the disclosure have been shown in the drawings, it is not intended that the disclosure be limited thereto, as it is intended that the disclosure be as broad in scope as the art will allow and that the specification be read likewise. For example, the knife limit buttons described herein may be employed for use with open surgical instrumentation in a similar manner as described with reference to the endoscopic instrumentation. Therefore, the above description should not be construed as limiting, but merely as exemplifications of particular embodiments. Those skilled in the art will envision other modifications within the scope and spirit of the claims appended hereto.

It will be understood that various modifications may be made to the aspects and features disclosed herein. Therefore, the above description should not be construed as limiting, but merely as exemplifications of various aspects and features. Those skilled in the art will envision other modifications within the scope and spirit of the claims appended thereto.

The invention may be described by reference to the following numbered paragraphs:-
1. A knife limit for a surgical instrument, comprising:
   a housing including a shaft extending therefrom configured to support an end effector at a distal end thereof, the end effector including first and second jaw members, at least one of the first or second jaw members moveable relative to the other jaw member to grasp tissue therebetween, at least one of the first or second jaw members including a knife channel defined therein and extending therealong from a proximal portion to a distal portion of the at least one jaw member;
   a knife assembly disposed within the housing and cooperating with a trigger disposed on the housing to translate a knife within the knife channel to the distal portion of the at least one jaw member upon actuation thereof; and
   a knife limit button operably disposed within the housing and configured to limit the distal translation of the knife within the knife channel upon selective actuation thereof, the knife limit button selectively movable between a first position allowing full translation of the knife within the knife channel to the distal portion upon actuation of the trigger and at least one second position limiting distal translation of the knife within the knife channel to a position proximal to the distal portion.
2. The knife limit for a surgical instrument according to paragraph 1 wherein the knife limit button is moveable to a second position wherein the knife limit button limits distal translation of the knife assembly which, in turn, limits distal translation of the knife within the knife channel.
3. The knife limit for a surgical instrument according to paragraph 1 wherein the knife limit button is moveable to a second position wherein the knife limit button limits proximal translation of the trigger which, in turn, limits distal translation of the knife within the knife channel.
4. The knife limit for a surgical instrument according to paragraph 1 wherein the knife limit button is rotatable to a second position wherein the knife limit button limits distal translation of the knife assembly which, in turn, limits distal translation of the knife with the knife channel.
5. The knife limit for a surgical instrument according to paragraph 1 wherein the knife assembly includes a cuff that rides atop the shaft and wherein upon actuation of the knife limit button the cuff is prevented from fully translating atop the shaft.
6. The knife limit for a surgical instrument according to paragraph 4 wherein the knife assembly includes a cuff that rides atop the shaft and wherein upon rotation of the knife limit button the cuff is prevented from fully translating atop the shaft.
7. The knife limit for a surgical instrument according to paragraph 1 further comprising a sensor operably coupled to the housing, the sensor configured to cooperate with the knife limit button to limit distal translation of the knife.
8. The knife limit for a surgical instrument according to paragraph 1 further comprising a sensor operably associated with the housing or a source of electrosurgical energy, the sensor configured to notify a user of the surgical instrument to actuate the knife limit button to limit distal translation of the knife.
9. A method of limiting distal translation of a knife blade of a robotic surgical instrument, comprising:
   selectively engaging an end effector onto a housing of a robotic surgical instrument and coupling the end effector to a jaw drive input, the end effector including first and second jaw members, at least one of the first or second jaw members moveable relative to the other jaw member to grasp tissue therebetween, at least one of the first or second jaw members including a knife channel defined therein and extending therealong from a proximal portion to a distal portion of the at least one jaw member;
   communicating with the end effector to recognize the end effector and associated operating parameters and characteristics therewith and communicating operational data back to an EPROM or PCB, the operational characteristics including a length of the knife channel defined within at least one of the jaw members of the end effector assembly;
   initiating a homing algorithm to determine a fully retracted or home position of a knife blade disposed within the knife channel between the jaw members and calculating a number of rotations of a knife drive coupler to fully translate the knife to the distal portion of the knife channel; and
   selectively actuating a knife limit button to limit the number of rotations of the knife drive coupler to limit distal translation of the knife within the knife channel.
10. The method of limiting distal translation of a knife blade of a robotic surgical instrument according to paragraph 9 further comprising selectively actuating the knife limit button based on feedback from a tissue sensor.
11. The method of limiting distal translation of a knife blade of a robotic surgical instrument according to paragraph 9 wherein the knife limit button operably communicates with a tissue sensor disposed within the housing or an electrosurgical energy source to determine automatic actuation of the knife limit button.
12. The method of limiting distal translation of a knife blade of a robotic surgical instrument according to paragraph 9 wherein the knife limit button operably communicates with a tissue sensor disposed within the housing or an electrosurgical energy source to alert a surgeon to actuate the knife limit button.
13. The method of limiting distal translation of a knife blade of a robotic surgical instrument according to paragraph 11 wherein the tissue sensor is configured to determine the quality of a tissue seal between jaw members.
14. The method of limiting distal translation of a knife blade of a robotic surgical instrument according to paragraph 12 wherein the tissue sensor is configured to determine the quality of a tissue seal between jaw members.
15. The method of limiting distal translation of a knife blade of a robotic surgical instrument according to paragraph 11 wherein the tissue sensor is configured to determine the quality of a tissue seal between jaw members based on at least one of the angular position of the jaw members, the tissue thickness, the activation time of the electrosurgical energy, or end tissue thickness after seal.
16. The method of limiting distal translation of a knife blade of a robotic surgical instrument according to paragraph 12 wherein the tissue sensor is configured to determine the quality of a tissue seal between jaw members based on at least one of the angular position of the jaw members, the tissue thickness, the activation time of the electrosurgical energy, or end tissue thickness after seal.

## Claims

1. A knife limit for a surgical instrument, comprising:
a housing including a shaft extending therefrom configured to support an end effector at a distal end thereof, the end effector including first and second jaw members, at least one of the first or second jaw members moveable relative to the other jaw member to grasp tissue therebetween, at least one of the first or second jaw members including a knife channel defined therein and extending therealong from a proximal portion to a distal portion of the at least one jaw member;
a knife assembly disposed within the housing and cooperating with a trigger disposed on the housing to translate a knife within the knife channel to the distal portion of the at least one jaw member upon actuation thereof; and
a knife limit button operably disposed within the housing and configured to limit the distal translation of the knife within the knife channel upon selective actuation thereof, the knife limit button selectively movable between a first position allowing full translation of the knife within the knife channel to the distal portion upon actuation of the trigger and at least one second position limiting distal translation of the knife within the knife channel to a position proximal to the distal portion.

2. The knife limit for a surgical instrument according to claim 1 wherein the knife limit button is moveable to a second position wherein the knife limit button limits distal translation of the knife assembly which, in turn, limits distal translation of the knife within the knife channel.

3. The knife limit for a surgical instrument according to claim 1 or claim 2 wherein the knife limit button is moveable to a second position wherein the knife limit button limits proximal translation of the trigger which, in turn, limits distal translation of the knife within the knife channel.

4. The knife limit for a surgical instrument according to any preceding claim wherein the knife limit button is rotatable to a second position wherein the knife limit button limits distal translation of the knife assembly which, in turn, limits distal translation of the knife with the knife channel.

5. The knife limit for a surgical instrument according to any preceding claim wherein the knife assembly includes a cuff that rides atop the shaft and wherein upon actuation of the knife limit button the cuff is prevented from fully translating atop the shaft.

6. The knife limit for a surgical instrument according to claim 4 wherein the knife assembly includes a cuff that rides atop the shaft and wherein upon rotation of the knife limit button the cuff is prevented from fully translating atop the shaft.

7. The knife limit for a surgical instrument according to any preceding claim further comprising a sensor operably coupled to the housing, the sensor configured to cooperate with the knife limit button to limit distal translation of the knife.

8. The knife limit for a surgical instrument according to any preceding claim further comprising a sensor operably associated with the housing or a source of electrosurgical energy, the sensor configured to notify a user of the surgical instrument to actuate the knife limit button to limit distal translation of the knife.

9. A method of limiting distal translation of a knife blade of a robotic surgical instrument, comprising:
selectively engaging an end effector onto a housing of a robotic surgical instrument and coupling the end effector to a jaw drive input, the end effector including first and second jaw members, at least one of the first or second jaw members moveable relative to the other jaw member to grasp tissue therebetween, at least one of the first or second jaw members including a knife channel defined therein and extending therealong from a proximal portion to a distal portion of the at least one jaw member;
communicating with the end effector to recognize the end effector and associated operating parameters and characteristics therewith and communicating operational data back to an EPROM or PCB, the operational characteristics including a length of the knife channel defined within at least one of the jaw members of the end effector assembly;
initiating a homing algorithm to determine a fully retracted or home position of a knife blade disposed within the knife channel between the jaw members and calculating a number of rotations of a knife drive coupler to fully translate the knife to the distal portion of the knife channel; and
selectively actuating a knife limit button to limit the number of rotations of the knife drive coupler to limit distal translation of the knife within the knife channel.

10. The method of limiting distal translation of a knife blade of a robotic surgical instrument according to claim 9 further comprising selectively actuating the knife limit button based on feedback from a tissue sensor.

11. The method of limiting distal translation of a knife blade of a robotic surgical instrument according to claim 9 or claim 10 wherein the knife limit button operably communicates with a tissue sensor disposed within the housing or an electrosurgical energy source to determine automatic actuation of the knife limit button.

12. The method of limiting distal translation of a knife blade of a robotic surgical instrument according to any of claims 9 to 11 wherein the knife limit button operably communicates with a tissue sensor disposed within the housing or an electrosurgical energy source to alert a surgeon to actuate the knife limit button.

13. The method of limiting distal translation of a knife blade of a robotic surgical instrument according to claim 12 wherein the tissue sensor is configured to determine the quality of a tissue seal between jaw members.

14. The method of limiting distal translation of a knife blade of a robotic surgical instrument according to any of claims 9 to 13 wherein the tissue sensor is configured to determine the quality of a tissue seal between jaw members based on at least one of the angular position of the jaw members, the tissue thickness, the activation time of the electrosurgical energy, or end tissue thickness after seal.
